# EUROPEAN PATENT SPECIFICATION

(11) **EP 0 961 590 B1**
(45) Date of publication and mention of the grant of the patent: **03.12.2003**
(21) Application number: 96935651.8
(22) Date of filing: 20.09.1996
(51) Int. Cl.: A61B 19/00

(54) **SURGICAL EXTENSION ARRANGEMENT FOR THE EXTENSION AND EXPANSION OF TISSUE**
CHIRURGISCHE ERWEITERUNGSVORRICHTUNG ZUM ERWEITERN UND AUSDEHNEN VON GEWEBE
DISPOSITIF EXTENSEUR CHIRURGICAL DESTINE A L'EXTENSION ET A L'ELARGISSEMENT DE TISSUS

(30) Priority: 11.10.1995 SE 9503530
(43) Date of publication of application: 08.12.1999
(73) Proprietor: Stretchex AB, 439 92 Onsala (SE)
(72) Inventor: HELLSTRÖM, Hans, S-439 92 Onsala (SE); BLOMQVIST, Gunnar, S-441 60 Alingsas (SE)
(74) Representative: Bergquist, Kjell Gunnar
(86) International application number: SE9601163
(87) International publication number: WO97013450

(56) References cited:
- EP-A- 0 279 534
- WO-A-92/15251
- WO-A-93/21849
- WO-A-95/08947
- US-A- 5 127 412
- US-A- 5 372 146
- DERWENT'S ABSTRACT, No. 88-196633/28, Week 8828; & SU,A,1 360 705 (KEMER MEDICINE INST), 23 December 1987.
- DERWENT'S ABSTRACT, No. 91-56408/08, Week 9108; & SU,A,1 553 073 (LASER SURGERY RES), 30 March 1990.

## Description

### TECHNICAL FIELD:

The present invention relates to a surgical extension arrangement for the extension and expansion of tissue, for example skin tissue. It is especially applicable when, for example, a skin blemish is to be removed and the surrounding skin shall be extended to allow sewing together of the cut edges so that the defect can be covered and to close skin defects which have arisen, for instance in the case of trauma.

### PRIOR ART:

When different skin defects have occurred, replacement tissue is often required. A large problem with such surgical operations is to acquire sufficient healthy skin to replace the defect. Skin may, for example, be taken from the upper leg of a human being and grafted. However, if skin is taken from such a healthy part of the body, it is usually not possible to take the whole skin but only the outer part thereof and the graft will therefore not be a complete replacement. The grafted skin is thinner and lacks the lower layer. The reason that it is not possible to remove complete skin from a healthy part is that the skin at this place must be allowed to grow back to its original shape.

There are, however, methods for bringing about a satisfactory replacement material, for example skin, when required. The body itself is allowed to produce this skin by means of a tissue expansion of the skin being brought about at a suitable place. This expansion is obtained by, most often under anaesthetic, inserting an expanding prosthesis, i.e. a silicone balloon having a filling nipple, under the skin and slowly filling the balloon with a brine solution so that an expansion and growth of the skin is obtained. To obtain a sufficient amount of skin, the bubble must be filled with new liquid via a filling valve inserted by operation, at certain intervals until the desired expansion has been obtained. This involves a visit to the surgeon every week for several months. This method for expanding of tissue is called the Radovan tissue expansion method and was first used during the seventies. When a sufficient amount of skin has been formed, the defect skin or the blemish is taken away and the expanded skin is moved into the defect and is sewn in place after removal of the expanding prosthesis. This is a demanding method of making replacement tissue which means that the patient has to be anaesthetized on two occasions and in the interval must visit the doctor once a week for several months. An example of this method is described in the European Patent Application 0 324 234.

Other methods for stretching of skin are known. For example, through the European Patent Application 0 279 534 it is known to use a band which is fastened with a device similar to a safety pin at both ends and at the edges of the skin pieces that are to be stretched. The band may be displaced through one of the fastening means as the skin is stretched so that all the time the required amount of stretching is obtained until the distance between the skin pieces has become so small that they can be sewn together. The fastening means are threaded into the skin approximately in the same way as a safety pin is fastened to a piece of cloth or the fastening means may be provided with hooks which go down into the skin. However, this stretching device is intended to stretch during a shorter time, for example 3-4 minutes, during several periods if so desired. The arrangement is complicated and painful to use.

Other examples of such stretching devices are disclosed in the US patents 3,926,193, 3,971,384 and 5,127,412. A further stretching device for stretching tissue has been proposed in the international patent application WO 92/15251. This application describes an arrangement for stretching skin and other tissue and consists of two fastening means for bands or threads wherein at least one of the fastening means is provided with a one-way locking for the thread. This thread can then be drawn only one way through the fastening means and it is therefore important that the correct tensioning force is applied.

The features of the preamble of claim 1 are known from the document WO-A-9 321 849.

### TECHNICAL PROBLEM:

The above methods and arrangements may function well as such but they are all connected with different disadvantages. Thus, the arrangements require an accurate setting of the tensioning force of the threads and this tensioning force may be difficult to calculate and it must be applied with complicated means. At one and the same cut edge of the skin, which edge may be very long, different strengths in the threads are also needed since the thickness and the quality of the skin may be different along the cut and especially may vary on different locations on the body and the cut may also be of such a shape that requires bendable or elastic fastening means.

### THE SOLUTION:

According to the present invention, the above problems connected to the known means have been solved and a surgical extension arrangement for the extension and expansion of tissue, especially, skin tissue, has been provided comprising the features of claim 1.

According to the invention, it is suitable that the friction locking is obtained by inserting the thread into a slot in the fastening device.

The fastening device according to the invention may suitably be elongated having a joint coupling, preferably in the shape of a ball in one end and a recess, preferably in the shape of a spherical cavity in the other end so that two or more fastening devices may be hingeably coupled to each other.

According to the invention the fastening device is suitably substantially cylindric and the slot opening runs in a plane perpendicular to the cylinder axis radially inwardly from the cylinder surface approximately to the cylinder axis and follows this in a plane parallel with the cylinder axis to approximately the axial middle part of the fastening device.

According to the invention the slot openings and the sizes of the thread/band diameters should be adapted to each other so that a predetermined friction and thereby a predetermined strain in the thread/band arises.

According to the invention, it is possible that fastening devices which give different friction in the friction locking are coupled to each other.

The invention also comprises a surgical needle for use together with the extension arrangement, which needle comprises an elastic and extensible thread fastened to that end of the needle which does not have a sharp tip.

### DESCRIPTION OF THE FIGURES:

The invention will now be described more in detail with reference to the attached drawings where
- Fig. 1: shows a unit of the fastening device in two views where the devices are turned at an angle of 90° in relation to each, other and where
- Fig. 2: shows a fastening device according to Fig. 1 coupled to other fastening devices so that a hinged row is created.

### DETAILED DESCRIPTION

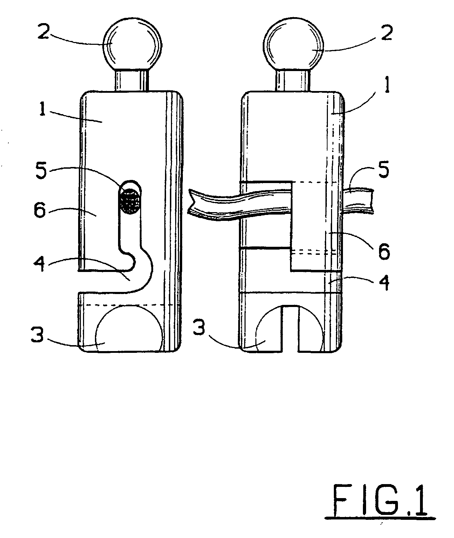

In Fig. 1 two fastening devices are shown which are turned at an angle of 90° in relation to each other. The fastening devices 1 are substantially cylindric and are shaped with a ball 2 in one end and a spherical cavity 3 in the other end. The fastening device 1 can hence be coupled together by pushing the ball 2 in one fastening device into the spherical cavity 3 in another fastening device.

As is best shown on the left fastening device 1 in Fig. 1, the cylinder body is provided with a slot 4 which runs in a plane which is angled 90° to the cylinder axis, which slot starts in the cylinder surface and extends approximately to the cylinder axis whereupon it extends in an axial direction and has an extension in a plane parallel with the cylinder axis. The slot extends approximately to the axially middle part of the cylinder.

This slot 4 is intended to house and clamp a thread 5 which is inserted into the slot. The slot 4 is made somewhat larger in the plane perpendicular to the shaft than in the plane parallel with the shaft to facilitate the insertion of the thread. The thread 5 will, as is best shown to the right on Fig. 1, to be clamped between a lug 6 and the main body of the fastening device 1. As is shown to the right on Fig. 1, this lug 6 is cut to approximately half of its width for practical reasons. The thread 5, which has a larger diameter than the width of the slot 4 at its bottom, will accordingly be maintained by friction of the fastening device 1. The larger the diameter of the thread 5 is compared to the width of the slot 4, the greater friction and the stronger grip will be created.

When the extension arrangement according to the invention is to be used, the surgeon first introduces the thread 5, which may be attached at one end to a surgical needle, in through the skin and under this as long as is deemed necessary, whereupon the thread is pushed up through the skin surface which is opposed to the first skin surface. Thereafter, the surgeon pushes the thread 5 into the respective slots of each fastening device 1 and he pulls the thread with such a force as is practically suitable. The thread 5 which is elastic will thereby obtain a contracting tension. How great this tension will be depends on the friction between the thread 5 and the slot 4. If the thread has been pulled out through the slot too much and has consequently received too great a tension, it will contract as much as the friction locking allows. It will therefore not be necessary for the surgeon to apply this stretching arrangement with any great accuracy.

The friction of the friction locking is partly determined by the material in the fastening device 1 and the thread 5 as well as the diameter of the thread 5 compared to the opening of the slot 4. The material in the thread 5 may for example be elastic silicone rubber. The fastening device 1 consists suitably of plastic material, such as polyethylene plastic.

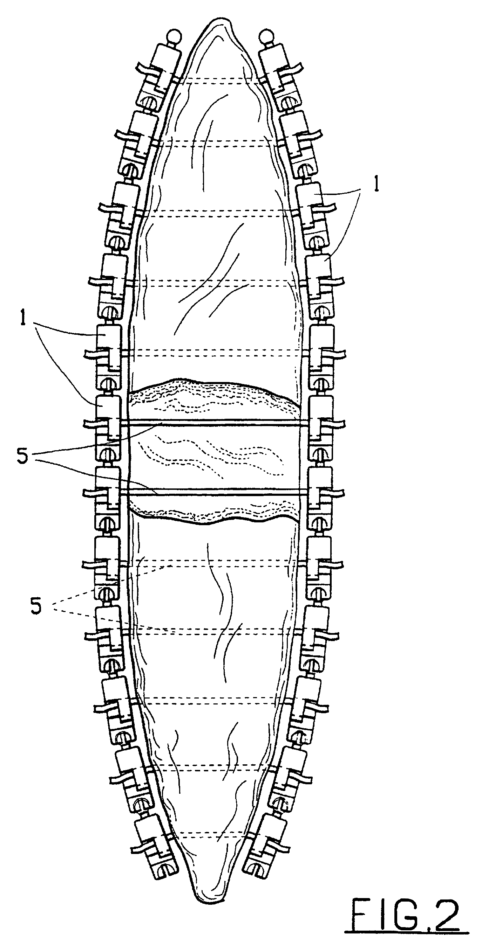

Fig. 2 shows two rows of fastening devices 1 which are coupled to each other and arranged on each side of an open cut. The two rows of fastening devices 1 are pulled towards each other by means of the elastic threads 5 which have been introduced between the rows transversely of these. The skin outside the fastening devices will therefore be pulled together with the force allowed by the friction locking of the fastening devices and the threads 5. As can be seen in the figure, the fastening devices 1 may be directed in different ways in relation to each other so that the whole row can be adapted to the cut edge of the skin.

It may be suitable that some threads 5 are tensioned more than other threads and another degree of the friction between the thread 5 and the slot 4 can then be chosen for these threads. Accordingly, it is possible to have different diameters of the threads or different openings in the slots 4. This may be particularly advantageous in the case of very long cuts or when the skin changes from one kind of skin to another along the cut.

Other materials than those mentioned above may also be applicable. However, a condition for this is that they can be sterile and that they are bio-compatible. The example above has been described using a thread 5 but also a band or the like can be used.

Through the present invention a very simple and functionally reliable extension arrangement for the extension and expansion of tissue has been brought about, which arrangement makes it easier for the surgeon to apply it and which easily can be adapted to give the extension required in each case and at each skin area. The example above has been described with two rows of fastening devices 1 but it is also conceivable to use only one row and that the second thread end is attached in some other way than through a row of fastening devices 1.

The invention is not limited to the above-described embodiment but can be varied in different ways within the scope of the claims.

## Claims

1. Surgical extension arrangement for the extension and expansion of tissue, such as skin tissue, comprising at least one elastic thread (5) or an elastic band and two opposing fastening devices (1) for the thread (5) or the band, the fastening devices (1) being of the same or different kind,
**characterized in that** the thread(s) (5) or the band is (are) fastened in the fastening devices (1) by friction locking.

2. Surgical extension arrangement according to claim 1, **characterized in that** the friction locking consists of the thread (5) being clamped in a slot (4) in the fastening device (1).

3. Surgical extension arrangement according to claim 1 or 2, **characterized in that** the fastening device 1 is elongated having a joint coupling preferably in the shape of a ball (2) in one end and a recess preferably in the shape of a spherical cavity (3) in the other end so that two or more fastening devices can hingedly be coupled to each other.

4. Surgical extension device according to any of claims 1-3, **characterized in that** the fastening device (1) is substantially cylindrical and that the slot opening (4) in a plane perpendicular to the cylinder axis extends radially inwardly from the cylinder surface approximately to the cylinder axis and follows this in a plane parallel with the cylinder axis to approximately the middle axial part of the fastening device (1).

5. Surgical extension device according to any of claims 1-4, **characterized in that** the diameter size of the slot openings (4) and of the thread/bands (5) are adapted to each other so that a predetermined friction and, depending thereon, a predetermined tensioning force will arise in the thread/band.

6. Surgical extension arrangement according to any of claims 1-5, **characterized in that** fastening devices (1) which give different friction in the friction locking are coupled to each other.

## Patentansprüche

1. Chirurgische Erweiterungsvorrichtung zum Erweitern und Ausdehnen von Gewebe, wie Hautgewebe, mit mindestens einem elastischen Faden (5) oder elastischem Band und mit zwei sich gegenüberliegenden Befestigungsvorrichtungen (1) für den Faden (5) oder das Band, wobei die Befestigungsvorrichtungen (1) von gleicher oder unterschiedlicher Art sind,
**dadurch gekennzeichnet,**
**dass** der Faden (die Fäden) (5) oder das Band an den Befestigungsvorrichtungen (1) durch Reibarretierung befestigt ist (sind).

2. Chirurgische Erweiterungsvorrichtung nach Anspruch 1,
**dadurch gekennzeichnet,**
**dass** die Reibarretierung durch Einklemmen des Fadens (5) in einen Schlitz (4) in der Befestigungsvorrichtung (1) gegeben ist.

3. Chirurgische Erweiterungsvorrichtung nach Anspruch 1,
**dadurch gekennzeichnet,**
**dass** die Befestigungsvorrichtung (1) länglich ist und an einem Ende ein Anschlussstück vorzugsweise in Gestalt einer Kugel (2) und am anderen Ende eine Aussparung vorzugsweise in Gestalt eines kugelförmigen Hohlraums (3) aufweist, so dass zwei oder mehr als zwei Befestigungselemente miteinander schwenkbeweglich verbindbar sind.

4. Chirurgische Erweiterungsvorrichtung nach einem der Ansprüche 1 bis 3,
**dadurch gekennzeichnet,**
**dass** die Befestigungsvorrichtung (1) im wesentlichen zylindrisch ist, und dass die Schlitzöffnung (4) sich in einer Ebene senkrecht zur Zylinderachse von der Zylinderoberfläche annähernd zur Zylinderachse radial nach innen erstreckt und von dort in einer Ebene parallel zur Zylinderachse annähernd bis zum Achsmittelabschnitt der Befestigungsvorrichtung (1) verläuft.

5. Chirurgische Erweiterungsvorrichtung nach einem der Ansprüche 1 bis 4,
**dadurch gekennzeichnet,**
**dass** die Durchmesser der Schlitzöffnungen (4) und der Fäden/Bänder (5) aneinander derartig angepasst sind, dass in dem Faden/Band eine vorbestimmte Reibung und von dieser abhängig eine vorbestimmte Zugkraft auftritt.

6. Chirurgische Erweiterungsvorrichtung nach einem der Ansprüche 1 bis 5,
**dadurch gekennzeichnet,**
**dass** Befestigungsvorrichtungen (1), die unterschiedliche Reibung in der Reibarretierung ergeben, miteinander verbunden sind.

## Revendications

1. Dispositif extenseur chirurgical destiné à l'extension et à l'élargissement de tissu, comme le tissu cutané, comprenant au moins un fil élastique (5) ou une bande élastique et deux dispositifs de fixation opposés (1) pour le fil (5) ou la bande, les dispositifs de fixation (1) étant identiques ou différents, **caractérisé en ce que** le ou les f i ls (5) ou la bande sont f ixés aux dispositifs de fixation (1) par verrouillage par friction.

2. Dispositif extenseur chirurgical selon la revendication 1, **caractérisé en ce que** le verrouillage par friction consiste **en ce que** le fil (5) est clampé dans une fente (4) dans le dispositif de fixation (1).

3. Dispositif extenseur chirurgical selon la revendication 1, **caractérisé en ce que** le dispositif de fixation (1) est allongé, ayant un couplage articulé de préférence sous la forme d'une balle (2) à une extrémité et d'un creux de préférence sous la forme d'une cavité sphérique (3) à l'autre extrémité de sorte que deux dispositifs de fixation ou plus puissent être couplés les uns aux autres par une charnière.

4. Dispositif extenseur chirurgical selon l'une quelconque des revendications 1 à 3, **caractérisé en ce que** le dispositif de fixation (1) est sensiblement cylindrique et **en ce que** l'ouverture de fente (4) dans un plan perpendiculaire à l'axe du cylindre s'étend radialement vers l'intérieur depuis la surface du cylindre approximativement jusqu'à l'axe du cylindre et suit celui-ci dans un plan parallèle à l'axe du cylindre jusqu'à approximativement la partie axiale médiane du dispositif de fixation (1).

5. Dispositif extenseur chirurgical selon l'une quelconque des revendications 1 à 4, **caractérisé en ce que** la taille de diamètre des ouvertures de fente (4) et des fils/bandes (5) sont adaptés les uns aux autres de sorte qu'une friction prédéterminée et, en fonction de celle-ci, une force de tension prédéterminée, sont provoquées dans le fil/la bande.

6. Dispositif extenseur chirurgical selon l'une quelconque des revendications 1 à 5, **caractérisé en ce que** des dispositifs de fixation (1) qui donnent une friction différente dans le verrouillage par friction sont couplés les uns aux autres.
